# EUROPÄISCHE PATENTSCHRIFT

(11) **EP 1 518 859 B1**
(45) Veröffentlichungstag und Bekanntmachung des Hinweises auf die Patenterteilung: **07.03.2007**
(21) Anmeldenummer: 04021686.3
(22) Anmeldetag: 13.09.2004
(51) Int. Cl.: C07D 495/04, C07D 333/32, C08G 61/12

(54) **Verfahren zur Reinigung von Thiophenen**
Process for the purification of thiophenes
Procédé de purification de thiophènes

(30) Priorität: 23.09.2003 DE 10343873
(43) Veröffentlichungstag der Anmeldung: 30.03.2005
(73) Patentinhaber: H.C. Starck GmbH & Co. KG, 38642 Goslar (DE)
(72) Erfinder: Brassat, Lutz, Dr., 51371 Leverkusen (DE); Kirchmeyer, Stephan, Dr., 51375 Leverkusen (DE)
(74) Vertreter: Clauswitz, Kai-Uwe Wolfram

(56) Entgegenhaltungen:
- EP-A- 0 937 721
- EP-A- 1 142 888
- WO-A-02/079295

## Beschreibung

Die Erfindung betrifft ein Verfahren zur Reinigung von bei Raumtemperatur flüssigen Thiophenen, die gemäß diesem Verfahren gereinigten Thiophene und deren Verwendung.

Thiophene werden beispielsweise zur Herstellung von leitfähigen Polymeren verwendet. Von besonderem Interesse sind hierbei Poly(3,4-alkylendioxythiophene), wie sie beispielsweise in EP-A 339 340 beschrieben sind. Diese Verbindungen zeichnen sich durch besondere Eigenschaften wie hohe Leitfähigkeit, hohe Transparenz und hervorragende Langzeitstabilität aus. Daher haben sie als organisch leitfähige Polymere in der Technik zunehmend Verwendung gefunden. So sind als wichtige Einsatzgebiete z.B. die Durchkontaktierung von Leiterplatten, die Antistatikausrüstung von photographischen Filmen und der Einsatz als Elektrode oder Feststoffelektrolyt in Feststoffelektrolytkondensatoren beschrieben.

Eine wichtige Voraussetzung bei der Herstellung der organischen leitfähigen Polymeren ist die hohe Reinheit der zu ihrer Herstellung notwendigen Ausgangsstoffe. Verunreinigungen, die in den Ausgangsstoffen enthalten sind, können die Polymerisation insofern negativ beeinflussen, dass die Polymerisation nicht, nur sehr langsam oder unvollständig erfolgt oder in unkontrolliertem Maße beschleunigt wird. In Folge dessen kann die Verarbeitungszeit dieser Monomere drastisch sinken, so dass diese in den Verarbeitungsprozessen nicht mehr einsetzbar sind.

Zusätzlich können die Eigenschaften der resultierenden Polymere ebenfalls negativ beeinflusst werden, indem die Verunreinigungen beispielsweise die Eigenfarbe des resultierenden Polymers negativ verändern und dadurch die Transparenz verschlechtert wird, die für den Einsatz der Polymere z.B. als transparente leitfähige oder antistatische Beschichtungen wesentlich ist.

Verunreinigungen, die ebenfalls polymerisationsfähig sind, können in das Polymer mit eingebaut werden und dadurch dessen Leitfähigkeit deutlich erniedrigen. Weitere nachteilige Auswirkungen von Verunreinigungen können sein, dass die Ordnung der leitfähigen Schichten durch Verunreinigungen erniedrigt werden kann, wodurch schlechtere Leitfähigkeiten resultieren, dass sich Verunreinigungen nach der Polymerisation auf der Oberfläche des Polymers anreichern und sich dadurch unerwünschte Übergangswiderstände ergeben, so dass die Funktion der leitfähigen Schicht eingeschränkt ist, oder aber dass die Langzeitstabilität der leitfähigen Polymere negativ beeinflusst wird, indem die Verunreinigungen beispielsweise die Reaktion des leitfähigen Polymers mit Sauerstoff initiieren und somit die Eigenschaften des Polymers signifikant verschlechtern.

Daher werden die zur Herstellung der organisch leitfähigen Polymeren notwendigen Ausgangsstoffe, die in der Regel durch chemische Umsetzungen aus Rohstoffen hergestellt werden, vor ihrem Einsatz gereinigt.

Dem Fachmann sind eine Reihe von Reinigungsoperationen bekannt, die zur Reinigung der Monomere für die Polymerisation zu organischen leitfähigen Polymeren grundsätzlich geeignet sind. Solche Reinigungsmethoden sind beispielsweise Destillation, Sublimation, Extraktion, Kristallisation, Chromatographie und Adsorption. Diese Reinigungsmethoden sind dem Fachmann seit langem bekannt und in gängigen Lehrbüchern beschrieben.

Bei Raumtemperatur flüssige Thiophene, die zur Herstellung von elektrisch leitfähigen Polymeren geeignet sind, sind aufgrund ihrer leichten Verarbeitbarkeit in der flüssigen Form von besonderer Bedeutung. Bei der Reinigung dieser Thiophene bieten sich dem Fachmann die Reinigungsmethoden an, die auf flüssige Stoffe anwendbar sind, vorzugsweise Destillation, die auch im großindustriellen Maßstab durchgeführt wird, Extraktion und Chromatographie.

Die destillative Reinigung von Thiophenen als Monomere für den Einsatz zur Herstellung von elektrisch leitfähigen Polymeren ist beispielsweise aus EP-A 1 142 888 bekannt. EP-A 1 142 888 lehrt, dass durch optimierte Reaktionsbedingungen die Anzahl und Menge an Nebenprodukten reduziert werden kann und z.B. 3,4-Ethylendioxythiophen in einer Reinheit von bis zu 97,7 % erhältlich ist. Jedoch lehrt EP-A 1 142 888 weiterhin, dass zur weiteren Reinigung eine zusätzliche Extraktion notwendig ist, um wasserlösliche Nebenprodukte zu entfernen und eine Reinheit von mehr als 99 % zu erzielen. Als Nebenkomponente, d.h. Verunreinigung, tritt bei dieser Synthese von 3,4-Ethylendioxythiophen überwiegend 3,4-Dimethoxythiophen auf.

Eine destillative Abtrennung von Verbindungen ist zudem nur dann möglich, wenn sich die zu trennenden Komponenten in ihrem Siedepunkt deutlich, d.h. mehr als 1°C unterscheiden. Je weniger sich die Siedepunkte unterscheiden, desto größer ist der apparative Aufwand zur Abtrennung, so dass derartige Trennungen wirtschaftlich nicht mehr durchzuführen sind. Da substituierte Thiophene, wie beispielsweise Alkylendioxythiophene vorzugsweise bei Unterdruck destilliert werden, wird der Unterschied der Siedepunkte weiter reduziert, was den Trennungsaufwand weiter erhöht.

Die Reinigung von 3,4-Alkylendioxythiophenen, insbesondere von 3,4-Ethylendioxythiophen, die mit 3,4-Dimethoxythiophen verunreinigt sind, stellt eine besondere Schwierigkeit dar. So kann das beispielsweise bei der Synthese von 3,4-Ethylendioxythiophen anfallende 3,4-Dimethoxythiophen aufgrund der sich nur um zwei Einheiten unterscheidende Molekülmasse und der sehr ähnlichen Struktur nur unter sehr hohem Aufwand abgetrennt werden, was die Aufreinigung über eine Destillation ab einem gewissen Reinheitsgrad nicht mehr wirtschaftlich macht. 3,4-Dimethoxythiophen als Verunreinigung hat jedoch den Nachteil, dass es bei Polymerisation mit in das Polymer eingebaut wird und so Eigenschaften des Polymeren, beispielsweise die Leitfähigkeit nachteilig beeinflussen kann.

Die chromatographische Reinigung von Thiophenen als Monomere für den Einsatz zur Herstellung von elektrisch leitfähigen Polymeren ist ebenfalls bekannt. WO-A 02/79295 beschreibt die Herstellung von flüssigen und festen chiralen Alkylendioxythiophenen und erwähnt in Beispielen die Reinigung durch eine Chromatographie an Siliziumdioxid. Die gemäß WO-A 02/79295 hergestellten Verbindungen weisen nach Reinigung eine Reinheit von bis zu 99,7 % auf. Die chromatographische Trennung ist aber ebenfalls mit Nachteilen behaftet. So sind zu Ihrer Durchführung große Mengen an Lösungsmitteln notwendig, da die zu trennenden Verbindungen in sehr verdünnter Form vorliegen müssen, um den gewünschten Trenneffekt zu erzielen. Weiterhin kann die chromatographische Trennung mit Hilfe einfacher Apparate nicht kontinuierlich betrieben werden, so dass jeweils nur kleine Mengen an gewünschtem aufgereinigtem Thiophen erhalten werden. Eine kontinuierliche Trennung großer Mengen wäre daher mit einem extrem hohen apparativen Aufwand verbunden, so dass eine solche Reinigung von Thiophenen wirtschaftlich nicht mehr durchführbar ist.

Die klassische Umkristallisation, bei der bei Raumtemperatur feste Thiophene bei erhöhter Temperatur, meist unter Rückfluss des Lösungsmittels in Lösung gebracht und anschließend durch Abkühlung wieder auskristallisiert werden, zur Reinigung von Thiophenen als Monomere für den Einsatzung zur Herstellung von elektrisch leitfähigen Polymeren ist ebenfalls bekannt und in WO-A 02/79295 beschrieben, beschränkt sich jedoch auf bei Raumtemperatur feste Thiophene.

Eine besondere Form der Kristallisation kann ebenfalls zur Kristallisation von flüssigen Thiophenen verwendet werden. Diese spezielle Form der Kristallisation, die Schmelzkristallisation, ist beispielsweise in N. Wynn, Chem. Engineering (1986), 93(8), 26-27 und in J. Ulrich und H. C Bülau, Editor(s): Myerson, Allan S. "Handbook of Industrial Crystallization (2nd Edition)" (2002), 161-179 beschrieben. Die Schmelzkristallisation beruht im wesentlichen darauf, dass man einen flüssigen Stoff bis zur Bildung einer Schmelze abkühlt, aus der nur der aufzureinigende Stoff auskristallisiert. Nach Kristallisation wird die im Idealfall alle Verunreinigungen enthaltende Mutterlauge abgetrennt. Gegebenenfalls wird der kristallisierte Stoff so leicht erhitzt, dass an dem Produkt anhaftende Verunreinigungen zusammen mit einem Teil des dann schmelzenden Stoffes entfernt werden können. Dieses Verfahren ist jedoch auf Stoffe bzw. Stoffgemische begrenzt, die größere Mengen an Verunreinigungen enthalten, welche in flüssiger Form abgetrennt werden können. Geringe Mengen von Verunreinigungen können über dieses Verfahren nur unwirtschaftlich entfernt werden, da große Mengen an gewünschter Verbindung mit abgetrennt werden müssen, um die kleine Menge an Verunreinigung herauszuwaschen. Zudem ist die Schmelzkristallisation bezüglich der Temperaturführung kritisch und damit apparativ aufwändig.

Es bestand daher weiterhin Bedarf an einem Verfahren zur Reinigung von bei Raumtemperatur flüssigen Thiophenen, bei dem eine extrem hohe Reinheit, vorzugsweise von mehr als 99,9 % erzielt wird, und welches die vorangehend beschriebenen Nachteile nicht aufweist.

Der vorliegenden Erfindung lag somit die Aufgabe zugrunde ein weniger aufwändiges Verfahren zur Reinigung von Thiophenen aufzufinden, mit welchem hochreine 3,4-Alkylendioxythiophene, vorzugsweise mit einer Reinheit von mehr als 99,9 %, hergestellt werden können.

Gegenstand der vorliegenden Erfindung ist ein Verfahren zur Reinigung von Thiophenen der allgemeinen Formel (I), 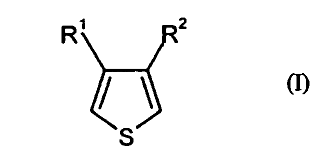 worin
- R¹ und R²: unabhängig von einander für Wasserstoff, für eine gegebenenfalls substituierte, gegebenenfalls durch 1 bis 5 Sauerstoff- und/oder Schwefelatome unterbrochene C₁-C₂₀-Alkylgruppe oder C₁-C₂₀-Oxyalkylgruppe oder gemeinsam für eine gegebenenfalls substituierte C₁-C₂₀-Dioxyalkylen- oder C₁-C₂₀-Dioxyarylengruppe stehen,
dadurch gekennzeichnet, dass ein bei Raumtemperatur flüssiges Thiophen der allgemeinen Formel (I) ausgewählt und aus einer Lösung in einem Lösungsmittel oder einem Lösungsmittelgemisch oder durch Einbringen in ein gekühltes Lösungsmitel oder Lösungsmittelgemisch oder in eine gekühlte Lösung des gleichen Thiophens bei einer Temperatur unterhalb der Schmelztemperatur des Thiophens als Feststoff ausgefällt und abgetrennt wird.

Unter bei Raumtemperatur flüssigen Thiophenen sind im Rahmen der Erfindung solche Thiophene zu verstehen, die ihren Schmelzpunkt unterhalb von + 40°C, bevorzugt unterhalb von + 30°C haben.

Raumtemperatur kann im Rahmen der Erfindung eine Temperatur von 10 bis 40°C, bevorzugt von 15 bis 30°C, besonders bevorzugt von 18 bis 25°C sein.

Bevorzugt werden mit dem erfindungsgemäßen Verfahren als Thiophene der allgemeinen Formel (I) Verbindungen der allgemeinen Formel (II), 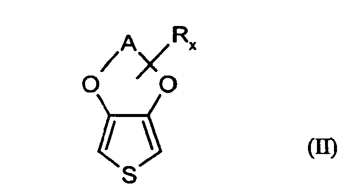 worin
- A: für einen gegebenenfalls substituierten C₁-C₅-Alkylenrest oder einen C₁-C₁₂-Arylenrest, bevorzugt für einen gegebenenfalls substituierten C₂-C₃-Alkylenrest, steht,
- R: für einen linearen oder verzweigten, gegebenenfalls substituierten C₁-C₁₈-Alkylrest, bevorzugt linearen oder verzweigten, gegebenenfalls substituierten C₁-C₁₄-Alkylrest, einen gegebenenfalls substituierten C₅-C₁₂-Cycloalkylrest, einen gegebenenfalls substituierten C₆-C₁₄-Arylrest, einen gegebenenfalls substituierten C₇-C₁₈-Aralkylrest, einen gegebenenfalls substituierten C₁-C₄-Hydroxyalkylrest, bevorzugt gegebenenfalls substituierten C₁-C₂-Hydroxyalkylrest, oder einen Hydroxylrest steht,
- x: für eine ganze Zahl von 0 bis 8 steht, bevorzugt von 0 bis 6, besonders bevorzugt für 0 oder 1 steht und
für den Fall, dass mehrere Reste R an A gebunden sind, diese gleich oder unterschiedlich sein können,
aufgereinigt.

Die allgemeine Formel (II) ist so zu verstehen, dass der Substituent R x-mal an den Alkylen- oder Arylenrest A gebunden sein kann.

Bevorzugte Verbindungen der allgemeinen Formel (II) sind solche der allgemeinen Formel (IIa), 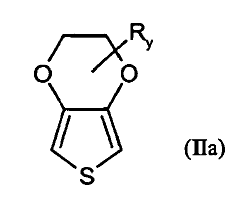 worin
- R: die in der allgemeine Formel (II) genannte Bedeutung hat und y für 0, 1, 2, 3 oder 4 steht.
C₁-C₅-Alkylenreste A sind im Rahmen der Erfindung Methylen, Ethylen, n-Propylen, n-Butylen oder n-Pentylen. C₁-C₁₂-Arylenreste A können im Rahmen der Erfindung beispielsweise Phenylen, Naphthylen, Benzyliden oder Anthracenyliden sein. C₁-C₁₈-Alkyl steht im Rahmen der Erfindung für lineare oder verzweigte C₁-C₁₈-Alkylreste wie beispielsweise Methyl, Ethyl, n- oder iso-Propyl, n-, iso-, sec- oder tert-Butyl, n-Pentyl, 1-Methylbutyl, 2-Methylbutyl, 3-Methylbutyl, 1-Ethylpropyl, 1,1-Dimethylpropyl, 1,2-Dimethylpropyl, 2,2-Dimethylpropyl, n-Hexyl, n-Heptyl, n-Octyl, 2-Ethylhexyl, n-Nonyl, n-Decyl, n-Undecyl, n-Dodecyl, n-Tridecyl, n-Tetradecyl, n-Hexadecyl oder n-Octadecyl. C₁-C₂₀-Alkylgruppen umfassen darüber hinaus beispielsweise n-Nonadecyl und n-Eicosyl. C₅-C₁₂-Cycloalkyl steht im Rahmen der Erfindung für C₅-C₁₂-Cycloalkylreste wie beispielsweise Cyclopentyl, Cyclohexyl, Cycloheptyl, Cyclooctyl, Cyclononyl oder Cyclodecyl, C₅-C₁₄-Aryl für C₅-C₁₄-Arylreste wie beispielsweise Phenyl oder Naphthyl, und C₇-C₁₈-Aralkyl für C₇-C₁₈-Aralkylreste wie beispielsweise Benzyl, o-, m-, p-Tolyl, 2,3-, 2,4-, 2,5-, 2,6-, 3,4-, 3,5-Xylyl oder Mesityl. C₁-C₂₀-Oxyalkyl steht im Rahmen der Erfindung für C₁-C₂₀-Oxyalkylreste wie beispielsweise Methoxy, Ethoxy, n- oder iso-Propoxy, n-, iso-, sec oder tert-Butoxy, n-Pentyloxy, 1-Methylbutyloxy, 2-Methylbutyloxy, 3-Methylbutyloxy, 1-Ethylpropyloxy, 1,1-Dimethylpropyloxy, 1,2-Dimethylpropyloxy, 2,2-Dimethylpropyloxy, n-Hexyloxy, n-Heptyloxy, n-Octyloxy, 2-Ethylhexyloxy, n-Nonyloxy, n-Decyloxy, n-Undecyloxy, n-Dodecyloxy, n-Tridecyloxy, n-Tetradecyloxy, n-Hexadecyloxy, n-Octadecyloxy, n- Nonadecyloxy oder n-Eicosyloxy. Die vorangehende Aufzählung dient der beispielhaften Erläuterung der Erfindung und ist nicht als abschließend zu betrachten. Als gegebenenfalls weitere Substituenten der Alkylen- oder Arylenreste A kommen zahlreiche organische Gruppen in Frage, beispielsweise Alkyl-, Cycloalkyl-, Aryl-, Halogen-, Ether-, Thioether-, Disulfid-, Sulfoxid-, Sulfon-, Sulfonat-, Amino-, Aldehyd-, Keto-, Carbonsäureester-, Carbonsäure-, Carbonat-, Carboxylat-, Cyano-, Alkylsilan- und Alkoxysilangruppen sowie Carboxylamidgruppen.

Sofern das aufzureinigende Thiophen ein oder mehrere Stereozentren aufweist, kann es sich bei dem Thiophen um ein Racemat, eine enantiomerenreine oder diastereomerenreine Verbindung oder um eine enantiomerenangereicherte oder diastereomerenangereicherte Verbindung handeln. Unter einer entantiomerenangereicherten Verbindung ist eine Verbindung mit einem Enantiomerenüberschuss (ee) von mehr als 50 % zu verstehen. Unter einer diastereomerenangereicherten Verbindung ist eine Verbindung mit einem Diastereomerenüberschuss (de) von mehr als 30 % zu verstehen. Es kann sich jedoch auch um eine beliebige Mischung aus Diastereomeren handeln.

Das aufzureinigende Thiophen weist vor Reinigung mit dem erfindungsgemäßem Verfahren bevorzugt eine Reinheit von größer 70 %, besonders bevorzugt eine Reinheit größer 90 % auf.

Die aufzureinigenden Thiophene der allgemeinen Formeln (I), (II) oder (IIa) sind nach dem Fachmann bekannten Verfahren herstellbar. Beispielsweise ist ein solches Herstellverfahren in EP-A 1 142 888 beschrieben.

Als Lösungsmittel werden solche eingesetzt, in denen sich das aufzureinigende Thiophen löst und welche einen ausreichend niedrigen Schmelzpunkt, vorzugsweise unterhalb von -40°C aufweisen. Beispielhaft seinen als geeignete Lösungsmittel Isobutylmethylketon, Chloroform, Methylenchlorid, Toluol, Methanol, Propanol, Ethanol, Aceton, iso-Propanol, n-Butanol, sec-Butanol, Dimethylformamid, Methyl-tert-butylether, Tetrahydrofuran, Diethylether, Hexan oder Pentan genannt.

Bevorzugte Lösungsmittel sind polare Lösungsmittel, besonders bevorzugt sind dabei Alkohole. Ganz besonders bevorzugt sind Methanol oder Ethanol.

Bei dem Lösungsmittel kann es sich um auch um ein Gemisch aus zwei oder mehreren Lösungsmitteln handeln.

Bevorzugt sind hierbei Mischungen aus einem oder mehreren Alkohol(en) gegebenenfalls mit einem oder mehreren weiteren Lösungsmittel(n). Hierzu ist es nicht zwingend erforderlich, dass jedes einzelne Lösungsmittel das Thiophen löst und einen entsprechend niedrigen Schmelzpunkt aufweist, sondern lediglich die Mischung muss diese Eigenschaften aufweisen. Besonders bevorzugt ist eine Mischung aus zwei Alkoholen, ganz besonders bevorzugt eine Mischung aus Methanol und Ethanol.

Das Lösungsmittel wird mit dem Thiophen in einem Verhältnis 0,01:1 bis 10:1, bevorzugt in einem Verhältnis von 0,3:1 bis 3:1 und ganz besonders bevorzugt in einem Verhältnis von 1:1 gemischt.

Das neue Verfahren wird z.B. so durchgeführt, dass man die aufzureinigenden Thiophene und wenigstens ein Lösungsmittel in beliebiger Reihenfolge zusammengibt, das oder die Lösungsmittel vor Zusammengeben mit dem Thiophen oder die während oder nach Zusammengeben erhaltene Lösung bis zu einer Temperatur abkühlt, bei der sich eine Mischung aus einem Feststoff und einer Flüssigkeit bildet, die Mischung aus einem Feststoff und einer Flüssigkeit gegebenenfalls nachrührt und anschließend den Feststoff abtrennt.

Bevorzugt werden das oder die Lösungsmittel vor Zusammengeben mit den Thiophenen oder die während oder nach Zusammengeben erhaltene Lösung bis zu einer Temperatur abkühlt, die mindestens 10°C, bevorzugt mindestens 20°C unterhalb der Schmelztemperatur des aufzureinigenden Thiophens in reiner Form liegt. Besonders bevorzugt wird auf 0°C oder niedriger, ganz besonders bevorzugt auf -15°C oder niedriger abgekühlt.

Das neue Verfahren kann beispielsweise so durchgeführt werden, dass das Thiophen in dem oder den Lösungsmittel(n) gelöst wird und dann diese Lösung mindestens so weit heruntergekühlt wird, dass das gereinigte Thiophen ausfällt bzw. auskristallisiert.

Hierbei kann das Thiophen in dem oder den Lösungsmittel(n) bei einer Temperatur oberhalb des Schmelzpunktes des Thiophens gelöst werden. Dabei ist eine Temperatur zwischen 0°C und + 40°C bevorzugt. Besonders bevorzugt ist eine Temperatur zwischen + 15°C und + 25°C.

Die erhaltene Lösung aus dem Lösungsmittel und dem Thiophen wird anschließend abgekühlt. Dabei wird die Lösung so weit abgekühlt, bis sich aus der Lösung das Thiophen in Form eines Feststoffes abscheidet bzw. auskristallisiert. Bevorzugt wird die Lösung auf eine Temperatur von mindestens 20°C unterhalb der Schmelztemperatur des reinen Thiophens abgekühlt. Besonders bevorzugt ist das Abkühlen auf -15°C oder auf eine Temperatur von niedriger als -15°C.

Die Lösung wird vorzugsweise mit einer Geschwindigkeit heruntergekühlt, dass das Thiophen innerhalb eines Zeitraumes von wenigen Minuten bis zu mehreren Stunden auskristallisiert. Bevorzugt ist dabei das Herunterkühlen auf die gewünschte Temperatur über einen Zeitraum von ca. einer Stunde.

Das Herunterkühlen kann durch eine Außenkühlung oder aber durch Einbringen eines inerten Kühlmediums bewirkt werden. Bevorzugt wird das Herunterkühlen durch Außenkühlung erzielt.

Während der Kühlphase scheidet sich aus der Lösung das Thiophen als Feststoff beispielsweise in Form von Kristallen ab. Dabei kann der erhaltene Feststoff das Thiophen als Reinstoff enthalten oder aus einem Gemisch des oder der Lösungsmittel(s) und dem Thiophen bestehen.

Alternativ kann das neue Verfahren so durchgeführt werden, dass das flüssige Thiophen in das bereits gekühlte Lösungsmittel, Lösungsmittelgemisch oder in eine gekühlte Thiophen-Lösung eindosiert wird.

Hierbei wird das Lösungsmittel auf eine Temperatur von mindestens 20°C unterhalb der Schmelztemperatur des reinen Thiophens abgekühlt. Besonders bevorzugt ist das Abkühlen auf -15°C oder eine Temperatur von niedriger als - 15°C.

Anschließend wird das flüssige Thiophen in das gekühlte Lösungsmittel - vorzugsweise über einen Zeitraum von wenigen Minuten bis zu mehreren Stunden - eindosiert. Die Dosiergeschwindigkeit ist dabei so zu wählen, dass das Thiophen nicht zu schnell ausfällt bzw. auskristallisiert und dabei Verunreinigungen in den Feststoff mit eingeschlossen werden. Bevorzugt ist eine Dosierzeit von mindestens 1 Stunde. Je nach Menge Thiophen, die zudosiert werden muss, können aber auch Dosierzeiten von weniger als einer Stunde ausreichen. Der erhaltene Feststoff kann ebenfalls das Thiophen als Reinstoff enthalten oder aus einem Gemisch des oder der Lösungsmittel(s) und dem Thiophen bestehen.

Bevorzugt wird die erhaltene Suspension anschließend über einen Zeitraum von 1 Minute bis zu 5 Stunden nachgerührt. Besonders bevorzugt ist hier bei eine Nachrührzeit von ca. drei Stunden.

Das Nachrühren wird bei einer Temperatur von mindestens 20°C unterhalb der Schmelztemperatur des reinen Thiophens durchgeführt. Bevorzugt ist dabei eine Temperatur von -15°C oder eine Temperatur von niedriger als -15°C.

Anschließend wird das ausgefällte bzw. auskristallisierte Produkt nach bekannten Methoden abgetrennt. Diese Abtrennung erfolgt bevorzugt durch eine Filtration. Die Filtration kann bei Normaldruck oder unter Druck durchgeführt werden.

Die Filtration erfolgt bevorzugt mit Hilfe eines temperierbaren Filteraggregates und wird so durchgeführt, dass das zu filtrierende Produkt während der Filtration als Feststoff vorliegt. Die Filtration wird bei einer Temperatur zwischen 0°C und -20°C durchgeführt. Bevorzugt wird die Filtration bei -15°C oder einer Temperatur von niedriger als -15°C durchgeführt.

Hiernach kann der erhaltene Feststoff mit einem oder mehreren Lösungsmittel(n) gewaschen werden um Reste von Verunreinigungen vom Filterkuchen zu entfernen. Hierzu verwendet man bevorzugt polare Lösungsmittel. Besonders bevorzugt werden Alkohole gegebenenfalls im Gemisch untereinander und/oder mit weiteren Lösungsmitteln verwendet. Besonders bevorzugt wird der Feststoff mit Ethanol oder Methanol oder einem Gemisch aus diesen gewaschen.

Für den Fall, dass der Filterkuchen zur Entfernung von am Filterkuchen anhafteten Verunreinigungsresten gewaschen wird, ist es sinnvoll, das Waschmittel, d.h. das zum Waschen verwendete Lösungsmittel, zu kühlen, um das Lösen von größeren Mengen gereinigten Thiophens im Waschmittel zu verhindern. Das Waschmittel hat beim Waschen eine Temperatur kleiner 0°C. Bevorzugt wird das Waschmittel zum Waschen auf -15°C oder niedriger heruntergekühlt.

Der dann erhaltene Feststoff wird auf eine Temperatur oberhalb des Schmelzpunktes des Thiophens über einen Zeitraum zwischen 5 Minuten und 5 Stunden erwärmt. Bevorzugt lässt man den Feststoff über einen Zeitraum von 1 Stunde aufschmelzen.

Nach dem Aufschmelzen kann der geschmolzene Feststoff noch Reste des vor der Kristallisation zugesetzten Lösungsmittels oder Reste des Waschmittels enthalten. Diese Reste lassen sich nach dem Fachmann bekannten Methoden z.B. durch einfache Destillation entfernen. Bei der Destillation wird das Lösungsmittel überdestilliert. Die Destillation kann bei Normaldruck oder unter vermindertem Druck durchgeführt werden. Bevorzugt wird sie bei vermindertem Druck bei Temperaturen zwischen 30°C und 150°C, bevorzugt zwischen 50°C und 100°C durchgeführt.

Das so erhaltene, im Sumpf verbleibende Thiophen weist nach dem vollständigen Abdestillieren des Lösungsmittels bevorzugt eine Reinheit von mindestens 99,50 %, bevorzugt mindestens 99,9 % auf. Beispielsweise enthalten Thiophene, die unter Verwendung von 3,4-Dimethoxythiophen synthetisiert wurden oder bei deren Synthese 3,4-Dimethoxythiophen als Nebenprodukt anfällt, nach Aufreinigung mit dem erfindungsgemäßen Verfahren weniger als 0,05 Gew.-% 3,4-Dimethoxythiophen. Ein derart niedriger Gehalt an 3,4-Dimethoxythiophen ist mit herkömmlichen Reinigungsverfahren, wie z.B. einfacher Destillation, nicht oder nur unter sehr hohem Ausbeuteverlust beim gewünschten Thiophen zu erzielen.

Alle Reinheitsangaben sind - sofern nicht anders erwähnt - Gewichtsprozentangaben.

Das nach der Destillation im Sumpf verbleibende Thiophen kann zur Abtrennung von farbgebenden Spuren ebenfalls überdestilliert werden. In der Regel erhält man dadurch ein für das Auge farbloses Thiophen. Die Destillation des Thiophens erfolgt ebenfalls bevorzugt bei Unterdruck.

Je nach verwendeter Menge Lösungsmittel im Verhältnis zur Menge eingesetzten Thiophens und je nach Temperatur bei der Ausfällung und gegebenenfalls beim Waschen können beispielsweise bis zu 70 %, vorzugsweise bis zu 90 %, besonders bevorzugt bis zu 95 % und ganz besonders bevorzugt nahezu 100 % des eingesetzten Thiophens in gereinigter Form erhalten werden. Der gegebenenfalls restliche Teil des eingesetzten Thiophens verbleibt in der Mutterlauge, d.h. z.B. im bei der Filtration abgetrennten Filtrat, oder gegebenenfalls im Waschmittel gelöst. Da eine Wiedergewinnung von nahezu 100 % des eingesetzten Thiophens in gereinigter Form wünschenswert ist, kann das Reinigungsverfahren in einer bevorzugten Ausführungsform auch so durchgeführt werden, dass die Mutterlauge einer vorangegangenen Fällung oder Kristallisation und/oder das Waschmittel als Lösungsmittel oder zusammen mit dem Lösungsmittel wieder in das Verfahren zur Reinigung von weiterem Thiophen eingesetzt werden.

Das erfindungsgemäße Verfahren ermöglicht die Aufreinigung von Thiophenen in einer einfachen Verfahrenweise. Die Produkte werden zudem in guter Ausbeute erhalten.

Die nach dem erfindungsgemäßen Verfahren gereinigten Thiophene eignen sich aufgrund ihrer hohen Reinheit hervorragend zur Herstellung von leitfähigen Polymeren oder zur Herstellung von organischen Halbleitern, die z.B. bei der Herstellung von Kondensatoren, Leiterplatten, antistatischen Schichten, transparenten leitfähigen Schichten, Displays, elektrochromen Verglasungen und integrierten Halbleiterschaltungen geeignet sind. Diese Verwendung ist weiterer Gegenstand der Erfindung.

Als Verbindungen, die mit dem erfindungsgemäßen Verfahren aufgereinigt werden können, seien beispielhaft folgende Verbindungen genannt:
3,4-Ethylendioxythiophen, 3,4-Methylendioxythiophen; R,S-3,4-(1'-Hydroxymethyl)ethylendioxythiophen, S-3,4-( 1'-Hydroxymethyl)ethylendioxythiophen; R-3,4-(1'-Hydroxymethyl)ethylendioxythiophen, 3,4-(2'-Hydroxy)propylendioxythiophen; 3,4-(1'-Methyl)ethylendioxythiophen, 3,4-(3'-tert.-Butyl)benzodioxythiophen; 3,4-( 1'-n-Hexyl)ethylendioxythiophen; 3,4-( 1'-Ethyl)-ethylendioxythiophen; 3,4-( 1'-n-Propyl)ethylendioxythiophen; 3,4-(1'-Butyl)ethylendioxythiophen; Thieno[3,4-b]-1,4-oxathiin; 3,4-Ethylendioxythiophen-1-methyl-N-methylcarbamat; 3,4-Ethylendioxythiophen-1-methyl-N-ethylcarbamat; 3,4-Ethylendioxythiophen-1-methyl-N-hexylcarbamat; 3,4-Ethylendioxythiophen-1-methyl-N-phenylcarbamat; 3,4-Ethylendioxythiophen-1-methyl-N-tolylcarbamat; (3,4-Ethylendioxythiophen-1-methyl)methylether; (3,4-Ethylendioxythiophen-1-methyl)ethylether; (3,4-Ethylendioxythiophen-1-methyl)propylether; (3,4-Ethylendioxythiophen-1-methyl)hexylether; 3-Hexylthiophen; 3 Octylthiophen

### Beispiele

### Beispiel 1:

### Reinigung von 3,4-Ethylendioxythiophen

1800 g 3,4-Ethylendioxythiophen mit einer Reinheit von 98,4 % und einem Gehalt an 3,4-Dimethoxythiophen von 0,3 % sowie leicht gelblicher Farbe wurden in einem Sulfierbecher mit 2400 ml Ethanol gerührt. Die Lösung wurde durch Außenkühlung auf eine Temperatur von -15°C heruntergekühlt und 3 h bei -15°C gerührt. Der entstandene Feststoff wurde mit Hilfe einer Nutsche abgetrennt und mit auf -15°C vorgekühltem Ethanol gewaschen. Der Filterkuchen wurde auf eine Temperatur von +20°C erwärmt. In einer Destillationsapparatur, bestehend aus einer Vorlagekolben, einer Destillationsbrücke und einem Kondensationskolben, wurde bei einem Druck von 16 hPa und einer Temperatur von 50°C zuerst das Lösungsmittel abdestilliert und anschließend bei einer Temperatur von 90°C und einem Druck von 16 hPa 3,4-Ethylendioxythiophen destilliert. Man erhielt 1374 g 3,4-Ethylendioxythiophen (76 % der Theorie) in einer Reinheit von 100 %. Das farblose Produkt wies kein 3,4-Dimethoxythiophen mehr auf.

### Beispiel 2:

### Reinigung von 3,4-Ethylendioxythiophen

1800 g 3,4-Ethylendioxythiophen mit einer Reinheit von 70 % und einem Gehalt an 3,4-Dimethoxythiophen von 0,3 % sowie dunkelbrauner Farbe wurden in einem Sulfierbecher mit 1800 g Ethanol gerührt. Die Lösung wurde durch Außenkühlung auf eine Temperatur von -23°C heruntergekühlt und 3 h bei -23°C gerührt. Der entstandene Feststoff wurde mit Hilfe einer Nutsche abgetrennt und mit auf -15°C vorgekühltem Ethanol gewaschen. Der abgetrennte Filterkuchen wurde auf eine Temperatur von +20°C erwärmt. In einer Destillationsapparatur, bestehend aus einer Vorlagekolben, einer Destillationsbrücke und einem Kondensationskolben, wurde bei einem Druck von 12 h Pa und einer Temperatur von 50°C zuerst das Lösungsmittel abdestilliert und anschließend bei einer Temperatur von 90°C und einem Druck von 12 hPa 3,4-Ethylendioxythiophen destilliert. Man erhielt 718 g 3,4-Ethylendioxythiophen (55 % d. Th.) in einer Reinheit von 99,2 %. Das Produkt wies kein 3,4-Dimethoxythiophen mehr auf.

## Patentansprüche

1. Verfahren zur Reinigung von Thiophen der allgemeinen Formel (1), 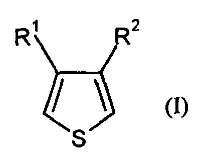 worin
R¹ und R² unabhängig von einander für Wasserstoff, für eine gegebenenfalls substituierte, gegebenenfalls durch 1 bis 5 Sauerstoff- und/oder Schwefelatome unterbrochene C₁-C₂₀-Alkylgruppe oder C₁-C₂₀-Oxyalkylgruppe oder gemeinsam für eine gegebenenfalls substituierte C₁-C₂₀-Dioxyalkylen- oder C₁-C₂₀-Dioxyarylengruppe stehen,
**dadurch gekennzeichnet, dass** ein bei Raumtemperatur flüssiges Thiophen der allgemeinen Formel (I), ausgewählt und durch Kühlen aus einer Lösung in einem Lösungsmittel oder Lösungsmittelgemisch oder durch Einbringung in ein gekühltes Lösungsmittel oder Lösungsmittelgemisch oder in eine gekühlte Lösung des gleichen Thiophens bei einer Temperatur unterhalb der Schmelztemperatur des Thiophens als Feststoff ausgefällt und abgetrennt wird, wobei ein bei Raumtemperatur flüssiges Thiophen ein solches mit einem Schmelzpunkt unterhalb von + 40°C ist.

2. Verfahren gemäß Anspruch 1, **dadurch gekennzeichnet, dass** als Thiophen der allgemeinen Formel (I) eine Verbindung der allgemeinen Formel (II) 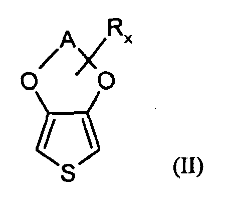 worin
A für einen gegebenenfalls substituierten C₁-C₅-Alkylenrest oder einen C₁-C₁₂-Arylenrest steht,
R für einen linearen oder verzweigten, gegebenenfalls substituierten C₁-C₁₈-Alkylrest, einen gegebenenfalls substituierten C₅-C₁₂-Cycloalkylrest, einen gegebenenfalls substituierten C₆-C₁₄-Arylrest, einen gegebenenfalls substituierten C₇-C₁₈-Aralkylrest, einen gegebenenfalls substituierten C₁-C₄-Hydroxyalkylrest oder einen Hydroxylrest steht,
x für eine ganze Zahl von 0 bis 8 steht und
für den Fall, dass mehrere Reste R an A gebunden sind, diese gleich oder unterschiedlich sein können,
eingesetzt wird.

3. Verfahren gemäß Anspruch 1 oder 2, **dadurch gekennzeichnet, dass** als Thiophen der allgemeinen Formel (I) eine Verbindung der allgemeinen Formel (IIa), 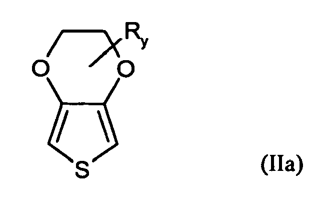 worin R die in Anspruch 2 genannte Bedeutung hat und y 0, 1, 2, 3 oder 4 bedeutet,
eingesetzt wird.

4. Verfahren gemäß wenigstens einem der Ansprüche 1 bis 3, **dadurch gekennzeichnet, dass** als Thiophen der allgemeinen Formel (I) eine Verbindung der allgemeinen Formel (IIa) eingesetzt wird, worin R die in Anspruch 2 genannte Bedeutung hat und y für 0 oder 1 steht.

5. Verfahren gemäß wenigstens einem der Ansprüche 1 bis 4, **dadurch gekennzeichnet, dass** als Lösungsmittel ein oder mehrere Alkohole gegebenenfalls im Gemisch mit einem oder mehreren weiteren von Alkoholen verschiedenen Lösungsmittel(n), bevorzugt Ethanol und/oder Methanol eingesetzt werden.

6. Verfahren gemäß einem der Ansprüche 1 bis 5, **dadurch gekennzeichnet, dass** zuerst das Lösungsmittel oder Lösungsmittelgemisch oder die Thiophen-Lösung vorgelegt und auf eine Temperatur abgekühlt wird, bei der sich bei Zugabe des flüssigen Thiophens eine Mischung aus festem Thiophen-Lösung bildet, und anschließend das flüssige Thiophen in das Lösungsmittel oder Lösungsmittelgemisch zugegeben wird.

7. Verfahren gemäß wenigstens einem der Ansprüche 1 bis 6, **dadurch gekennzeichnet, dass** eine Thiophen-Lösung oder ein Lösungsmittelvorlage vor der Zugabe des Thiophens auf eine Temperatur abgekühlt wird, die mindestens 20°C unterhalb des Schmelzpunktes des Thiophens (nach Formel (I) liegt.

8. Verfahren gemäß wenigstens einem der Ansprüche 1 bis 7, **dadurch gekennzeichnet, dass** die Abtrennung des festen Thiophens von der Lösung bei einer Temperatur erfolgt die mindestens 20°C unterhalb des Schmelzpunktes des Thiophens nach Formel (I) liegt.

9. Verfahren gemäß einem der Ansprüche 1 bis 8, **dadurch gekennzeichnet, dass** das im kristallisierten Thiophen ggf. verbliebene restliche Lösungsmittel durch einen weiteren Reinigungsschritt, insbesondere durch Destillation des Thiophens entfernt wird.

## Claims

1. Process for the purification of thiophenes of the general formula (I) 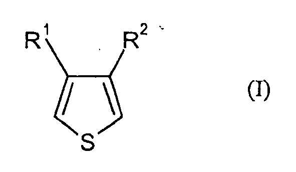 wherein
R¹ and R² independently of one another represent hydrogen, optionally substituted C₁-C₂₀-alkyl groups or C₁-C₂₀-oxyalkyl groups which are optionally interrupted by 1 to 5 oxygen and/or sulfur atoms, or together represent an optionally substituted C₁-C₂₀-dioxyalkylene or C₁-C₂₀-dioxyarylene group,
**characterized in that** a thiophene of the general formula (I) which is liquid at room temperature is chosen and is precipitated as a solid from a solution in a solvent or solvent mixture by cooling or by introduction into a cooled solvent or solvent mixture or into a cooled solution of the same thiophene at a temperature below the melting point of the thiophene and is separated off, a thiophene which is liquid at room temperature being one having a melting point of below +40 °C.

2. Process according to claim 1, **characterized in that** the thiophene of the general formula (I) employed is a compound of the general formula (II) 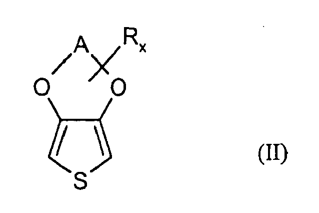 wherein
A represents an optionally substituted C₁-C₅-alkylene radical or a C₁-C₁₂-arylene radical,
R represents a linear or branched, optionally substituted C₁-C₁₈-alkyl radical, an optionally substituted C₅-C₁₂-cycloalkyl radical, an optionally substituted C₆-C₁₄-aryl radical, an optionally substituted C₇-C₁₈-aralkyl radical, an optionally substituted C₁-C₄-hydroxyalkyl radical or a hydroxyl radical,
x represents an integer from 0 to 8 and
in the case where several radicals R are bonded to A, these can be identical or different.

3. Process according to claim 1 or 2, **characterized in that** the thiophene of the general formula (I) employed is a compound of the general formula (IIa) 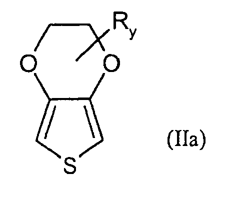 wherein R has the meaning given in claim 2 and y denotes 0, 1, 2, 3 or 4.

4. Process according to at least one of claims 1 to 3, **characterized in that** the thiophene of the general formula (I) employed is a compound of the general formula (IIa) wherein R has the meaning given in claim 2 and y represents 0 or 1.

5. Process according to at least one of claims 1 to 4, **characterized in that** one or more alcohols, optionally in a mixture with one or more further solvent(s) other than alcohols, preferably ethanol and/or methanol, are employed as the solvent.

6. Process according to one of claims 1 to 5, **characterized in that** the solvent or solvent mixture or the thiophene solution is first introduced into the purification vessel and cooled to a temperature at which, on addition of the liquid thiophene, a mixture of solid thiophene and solution forms, and the liquid thiophene is then added to the solvent or solvent mixture.

7. Process according to at least one of claims 1 to 6, **characterized in that** a thiophene solution or a solvent reservoir is cooled to a temperature which is at least 20°C below the melting point of the thiophene (according to formula (I) before the addition of the thiophene.

8. Process according to at least one of claims 1 to 7, **characterized in that** the solid thiophene is separated off from the solution at a temperature which is at least 20°C below the melting point of the thiophene according to formula (I).

9. Process according to one of claims 1 to 8, **characterized in that** the residual solvent which may remain in the crystallized thiophene is removed by a further purification step, in particular by distillation of the thiophene.

## Revendications

1. Procédé pour la purification de thiophène de la formule générale (I), 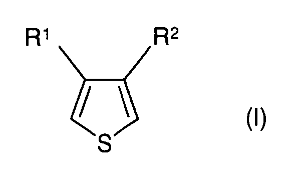 dans laquelle
R¹ et R² représentent indépendamment l'un de l'autre un atome d'hydrogène, un groupe alkyle en C₁-C₂₀ ou un groupe oxyalkyle en C₁-C₂₀ éventuellement substitué, éventuellement interrompu par de 1 à 5 atomes d'oxygène et/ou de soufre ou représentent ensemble un groupe dioxyalkylène en C₁-C₂₀ ou dioxyarylène en C₁-C₂₀ éventuellement substitué,
**caractérisé en ce que** l'on choisit un thiophène de la formule générale (I) liquide à température ambiante et on le fait précipiter et on le sépare comme une matière solide par refroidissement à partir d'une solution dans un solvant ou dans un mélange de solvant ou par introduction dans un solvant ou dans un mélange de solvant refroidi ou dans une solution refroidie du même thiophène à une température inférieure à la température de fusion du thiophène, un thiophène liquide à température ambiante est un thiophène avec un point de fusion inférieur à +40°C.

2. Procédé selon la revendication 1, **caractérisé en ce que** l'on utilise comme thiophène de la forme générale (I) un composé de la formule générale (II) 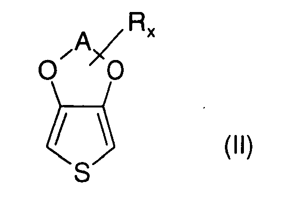 dans laquelle
A représente un reste alkylène en C₁-C₅ ou un reste arylène en C₁-C₁₂ éventuellement substitué,
R représente un reste alkyle en C₁-C₁₈ linéaire ou ramifié, éventuellement substitué, un reste cycloalkyle en C₅-C₁₂ éventuellement substitué, un reste aryle en C₆-C₁₄ éventuellement substitué, un reste aralkyle en C₇-C₁₈ éventuellement substitué, un reste hydroxyalkyle en C₁-C₄ éventuellement substitué ou un reste hydroxyle,
x représente un nombre entier de 0 à 8 et
dans le cas où plusieurs restes R sont liés à A, ceux-ci peuvent être identiques ou différents.

3. Procédé selon la revendication 1 ou 2, **caractérisé en ce que** l'on utilise comme thiophène de la formule générale (I) un composé de la formule générale (IIa), 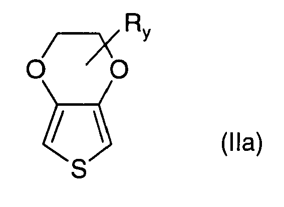 dans laquelle R a la signification citée dans la revendication 2 et y est égal à 0, 1, 2, 3 ou 4.

4. Procédé selon au moins l'une quelconque des revendications 1 à 3, **caractérisé en ce que** l'on utilise comme thiophène de la formule générale (I) un composé de la formule générale (IIa), où R a la signification citée dans la revendication 2 et y est égal à 0 ou 1.

5. Procédé selon au moins l'une quelconque des revendications 1 à 4, **caractérisé en ce que** l'on utilise comme solvant un ou plusieurs alcools éventuellement en mélange avec un ou plusieurs autre(s) solvant(s) différent(s) des alcools, de préférence l'éthanol et/ou le méthanol.

6. Procédé selon l'une quelconque des revendications 1 à 5, **caractérisé en ce que** l'on charge dans un premier temps le solvant ou le mélange de solvant ou la solution de thiophène et on refroidit à une température, à laquelle il se forme, par addition du thiophène liquide, un mélange à partir de la solution de thiophène solide et **en ce que** l'on introduit ensuite le thiophène liquide dans le solvant ou dans le mélange de solvant.

7. Procédé selon au moins l'une quelconque des revendications 1 à 6, **caractérisé en ce que** l'on refroidit une solution de thiophène ou une précharge de solvant avant l'addition du thiophène à une température qui est au moins 20°C inférieure au point de fusion du thiophène selon la formule (I).

8. Procédé selon au moins l'une quelconque des revendications 1 à 7, **caractérisé en ce que** l'on réalise la séparation du thiophène solide de la solution à une température qui est au moins 20°C inférieure au point de fusion du thiophène selon la formule (I).

9. Procédé selon au moins l'une quelconque des revendications 1 à 8, **caractérisé en ce que** l'on élimine le solvant résiduel restant éventuellement dans le thiophène cristallisé par une étape de purification supplémentaire, en particulier par distillation du thiophène.
